(19) Europäisches Patentamt
European Patent Office
Office européen des brevets

(11) **EP 4 421 742 B1**

(12) **EUROPÄISCHE PATENTSCHRIFT**

(45) Veröffentlichungstag und Bekanntmachung des Hinweises auf die Patenterteilung:
**15.07.2026 Patentblatt 2026/29**

(21) Anmeldenummer: **23188706.8**

(22) Anmeldetag: **31.07.2023**

(51) Internationale Patentklassifikation (IPC):
***G06T 12/10*** (2026.01)

(52) Gemeinsame Patentklassifikation (CPC):
**G06T 12/10; A61B 6/03; A61B 6/032; A61B 6/5205;** G06T 2211/436; G06T 2211/441

(54) **GENERIERUNG VON AUGMENTIERTEN CT-PROJEKTIONSDATEN VON EINEM UNTERSUCHUNGSOBJEKT**

GENERATION OF AUGMENTED CT PROJECTION DATA FROM AN OBJECT TO BE EXAMINED

GÉNÉRATION DE DONNÉES DE PROJECTION CT AUGMENTÉES À PARTIR D'UN OBJET EXAMINÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**28.08.2024 Patentblatt 2024/35**

(73) Patentinhaber: **Siemens Healthineers AG 91301 Forchheim (DE)**

(72) Erfinder:
• **Dr. Baer-Beck, Matthias**
**91080 Spardorf (DE)**
• **Dr. Zeller, Mario**
**91054 Erlangen (DE)**

(74) Vertreter: **Siemens Healthineers Patent Attorneys Postfach 22 16 34 80506 München (DE)**

(56) Entgegenhaltungen:
• **ADISHESHA AMOGH SUBBAKRISHNA ET AL: "Sinogram Domain Angular Upsampling of Sparse-View Micro-CT with Dense Residual Hierarchical Transformer and Noise-Aware Loss", BIORXIV, 12 May 2023 (2023-05-12), XP093121503, Retrieved from the Internet <URL:https://www.biorxiv.org/content/10.1101/2023.05.09.540072v1.full.pdf> [retrieved on 20240119], DOI: 10.1101/2023.05.09.540072**

• **CHRISTIANSEN CAROLYN ET AL: "Sinogram Interpolation Inspired by Single-Image Super Resolution.", JOURNAL OF BIOTECHNOLOGY AND ITS APPLICATIONS 2023, vol. 2, no. 1, 15 June 2023 (2023-06-15), XP002810863, ISSN: 2771-9014**

• **VASWANI ASHISH ET AL: "Attention Is All You Need", 31ST CONFERENCE ON NEURAL INFORMATION PROCESSING SYSTEMS NIPS 2017, 9 December 2017 (2017-12-09), Long Beach, CA, USA, XP055832424, Retrieved from the Internet <URL:https://papers.nips.cc/paper/2017/file/3f5ee243547dee91fbd053c1c4a845aa-Paper.pdf> [retrieved on 20210817]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## EP 4 421 742 B1

**Beschreibung**

Die Erfindung betrifft ein Verfahren zur Generierung von augmentierten CT-Projektionsdaten von einem Untersuchungsobjekt. Weiterhin betrifft die Erfindung ein Verfahren zum Generieren von CT-Bilddaten. Die Erfindung betrifft auch eine Projektionsdaten-Augmentierungseinrichtung. Weiterhin betrifft die Erfindung ein Computertomographiesystem.

In der Computertomographie werden dreidimensionale Aufnahmen eines Untersuchungsobjekts dadurch gewonnen, dass eine Röntgenquelle um das Untersuchungsobjekt rotiert und mittels kreisförmig angeordneter Detektor-Arrays zu jeweils anderen Winkelpositionen der Quelle eine Vielzahl an Projektionen aufgenommen werden, welche in einem Sinogramm angeordnet werden. Die Eigenschaften der Radon-Transformation bzw. des Fourier Slice-Theorems ermöglichen es, anhand der Projektionen die räumliche Verteilung der Schwächungswerte der Röntgenstrahlung zu ermitteln. Dies wird üblicherweise mit der sogenannten gefilterten Rückprojektion, in den letzten Jahren verstärkt auch mit iterativen Verfahren, erreicht.

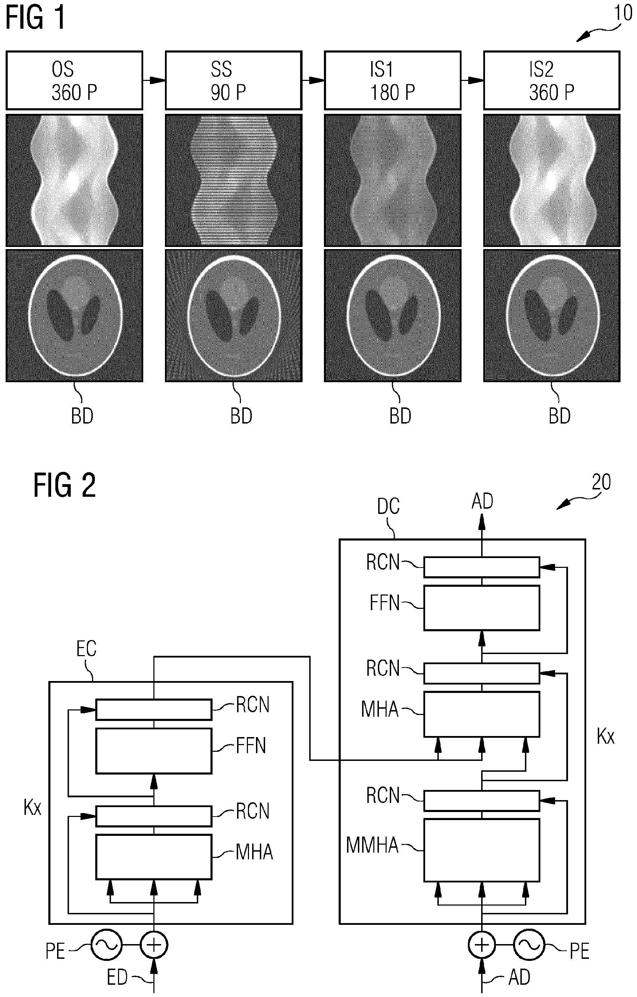

Ein Nachteil der CT-Bildgebung ergibt sich generell aus der durch die vielen Aufnahmen entstehenden hohen Strahlenbelastung. Ein Weg zur Reduktion der Strahlenbelastung besteht darin, die Anzahl der aufgenommenen Projektionen (Views) zu reduzieren. Eine zu geringe Anzahl an Einzelaufnahmen bzw. Projektionen führt jedoch zu Streaking-Artefakten bei der gefilterten Rückprojektion. Dies ist in FIG 1 dargestellt. Links ist das Sinogramm (oben) und die entsprechende Bildrekonstruktion (unten) mit 360 Views bzw. Projektionen dargestellt. Ein mit geringerer Winkelauflösung aufgenommenes Sinogramm (zweites von links) führt zu starken Streaking-Artefakten in der Bildrekonstruktion.

Eine verbesserte Rekonstruktion lässt sich erzielen, indem fehlende Linien eines Sinogramms interpoliert werden. Verschiedene mehr oder minder gut funktionierende Interpolationsverfahren sind in Kim et al., 2018, "Image enhancement for computed tomography using directional interpolation for sparsely-sampled sinogram", Optik 166(2018) 227-235 gegenübergestellt.

Kürzlich wurden Rekonstruktionen basierend auf neuronalen Netzen vorgeschlagen. Bei diesen neuartigen Rekonstruktionsverfahren werden zusätzliche Projektionen mittels Encoder-Decoder-Architekturen, insbesondere U-Net-Architekturen, interpoliert. Derartige Verfahren sind in Lee et al., "Deep-Neural-Network-Based Sinogram Synthesis for Sparse-View CT Image Reconstruction", IEEE Transactions on Radiation and Plasma Medical Sciences, Vol. 4, No. 2, March 2019 und in Chao et al., "Sparse-View Cone Beam CT Reconstruction using dual CNNs in Projection Domain and Image Domain", Neurocomputing 493 (2022) 536 - 547 beschrieben.

Als Stand der Technik sind ferner Adishesha Amogh Subbakrishna et al.: "Sinogram Domain Angular Upsampling of Sparse-View Micro-CT with Dense Residual Hierarchical Transformer and Noise-Aware Loss", bioRxiv, 12. Mai 2023, und Christiansen C, Zeng GL: "Sinogram Interpolation Inspired by Single-Image Super Resolution", Journal of biotechnology and its applications 2023, Bd. 2, Nr. 1, 15. Juni 2023, zu nennen.

Es ist somit eine Aufgabe, ein Verfahren im Zusammenhang mit der CT-Bildgebung zu entwickeln, mit dem eine verbesserte Bildqualität bei der CT-Bildgebung bei einer gleichbleibenden Anzahl von Projektionen oder bei gleichbleibender Auflösung des zur Aufnahme der Projektionen genutzten Röntgendetektors oder bei gleichbleibender Bildqualität eine Reduzierung der Anzahl der Projektionen und damit eine Reduzierung der Strahlenbelastung des abgebildeten Untersuchungsobjekts im Vergleich zu herkömmlichen Verfahren oder bei gleichbleibender Bildqualität eine Reduzierung des Datenstroms bzw. des Datentransfers von dem zur Aufnahme der Projektionen genutzten Röntgendetektor erreicht wird.

Diese Aufgabe wird durch ein Verfahren zur Generierung von augmentierten CT-Projektionsdaten von einem Untersuchungsobjekt gemäß Patentanspruch **1**, ein Verfahren zum Generieren von CT-Bilddaten gemäß Patentanspruch **8**, eine Projektionsdaten-Augmentierungseinrichtung gemäß Patentanspruch 9 und durch ein Computertomographiesystem gemäß Patentanspruch 10 gelöst.

Bei dem erfindungsgemäßen Verfahren zur Generierung von augmentierten CT-Projektionsdaten von einem Untersuchungsobjekt werden unterabgetastete CT-Projektionsdaten von dem Untersuchungsobjekt empfangen (auch als Schritt i) bezeichnet). Als Unterabtastung soll eine Messung mit einer reduzierten Messinformation verstanden werden. Eine solche Messinformation umfasst insbesondere eine reduzierte Anzahl von Projektionen und/oder eine reduzierte Auflösung, mit der ein Röntgendetektor CT-Projektionsdaten erfasst. "Reduziert" soll hier relativ zu einer vorbestimmten Referenzgröße verstanden werden. Eine solche Referenzgröße umfasst insbesondere eine technisch maximal mögliche Detailliertheit einer Messinformation. Diese maximale Größe wird bevorzugt durch die maximale Anzahl der Projektionen pro 360°- Umlauf der Röntgenquelle und des der Röntgenquelle gegenüberliegenden Röntgendetektors eines CT-Systems und/oder durch die Anzahl der Detektorpixel des Röntgendetektors in Zeilenrichtung und in Kanalrichtung festgelegt. In erster Linie reduziert sich durch eine Reduktion der Anzahl der Projektionen erst einmal die Auflösung der auf Basis der Projektionsdaten gewonnenen CT-Bilddaten. Der Übergang zwischen reduzierter Auflösung und dem Auftreten von Artefakten ist dann fließend. Unterschreitet man einen gewissen Punkt bei der Anzahl der aufgenommenen Projektionen pro 360°, so treten Strichartefakte auf.

Die CT-Projektionsdaten umfassen Projektionsmessdaten, die von einem Untersuchungsobjekt durch einen Messprozess gewonnen wurden. Die CT-Projektionsdaten können direkt von dem Untersuchungsobjekt durch ein Computertomographiesystem aufgenommen werden und anschließend mit dem erfindungsgemäßen Verfahren augmentiert werden. Die CT-Projektionsdaten können aber auch aus einer Datenbank und/oder über ein Datennetzwerk bezogen werden.

Weiterhin werden synthetische CT-Projektionsdaten durch Anwendung eines auf einer Transformer-Architektur basierenden KI-basierten Modells ("KI" ist ein Akronym für "Künstliche Intelligenz" und umfasst Verfahren, bei denen Algorithmen angewendet werden, die ähnliche Ergebnisse wie die Anwendung menschlicher Intelligenz erzielen können. Insbesondere fallen darunter Konzepte wie maschinelles Lernen, künstliche neuronale Netzwerke oder Deep Learning.) auf die unterabgetasteten CT-Projektionsdaten generiert (auch als Schritt ii) bezeichnet). Die Nutzung einer Transformer-Architektur zur Ermittlung von synthetischen CT-Projektionsdaten hat im Vergleich zu herkömmlichen Modellen, bei denen nur unmittelbar benachbarte Projektionen bzw. Projektionsdatensätze für die Ermittlung der synthetischen CT-Projektionsdaten genutzt werden, den Vorteil, dass auch CT-Projektionsdatensätze bei der Ermittlung der synthetischen CT-Projektionsdaten berücksichtigt werden, die nicht direkt benachbart sind. Vorzugsweise werden auch die um 180° gedreht aufgenommenen CT-Projektionsdatensätze bei der Generierung von synthetischen CT-Projektionsdaten bzw. CT-Projektionsdatensäten mitberücksichtigt. Allgemein formuliert, können Merkmale aus der gesamten Zeitserie eines CT-Projektionsdatensatzes für die Ermittlung der synthetischen CT-Projektionsdaten herangezogen werden. Vorteilhaft wird die Informationsgrundlage für die Ermittlung der synthetischen Projektionsdaten verbreitert.

Einzelheiten zur Transformer-Architektur sind in Vaswani A. et al., "Attention Is All You Need", 31st Conference on Neural Information Processing Systems (NIPS 2017), Long Beach, CA, USA beschrieben, welche Schrift durch Bezug mit in die vorliegende Patentanmeldung einbezogen ist.

Dabei werden zwischen den benachbarten CT-Projektionsdatensätzen der unterabgetasteten CT-Projektionsdaten liegende zusätzliche CT-Projektionsdatensätze durch Interpolation zwischen den benachbarten CT-Projektionsdatensätzen gewonnen (was ebenfalls Teil des Schritts ii) ist) bezeichnet). CT-Projektionsdatensätze umfasst CT-Projektionsdaten, welche jeweils genau einer einzigen Projektion entsprechen bzw. mit einer Messung in einem einzigen Winkel des Röntgendetektors aufgenommen wurden.

Schließlich werden augmentierte CT-Projektionsdaten durch verschachteltes Kombinieren der unterabgetasteten CT-Projektionsdaten und der synthetischen CT-Projektionsdaten gewonnen (auch als Schritt iii) bezeichnet). Als verschachteltes Kombinieren soll eine abwechselnde Anordnung der einzelnen Projektionsdatensätze der synthetischen CT-Projektionsdaten und der unterabgetasteten Projektionsdaten verstanden werden. Insbesondere sollen sich in einer besonders bevorzugten Ausgestaltung einzelne "Linien" der Sinogramme der synthetischen CT-Projektionsdaten und der unterabgetasteten Projektionsdaten bzw. die diesen Linien zugeordneten CT-Projektionsdaten bei deren verschachtelter Kombination abwechseln.

Wie bereits vorstehend angedeutet, kann zusätzlich oder alternativ auch eine niedrigere Detektorauflösung kompensiert werden, indem zwischen Projektionsdaten(-Teil)sätzen (auch Projektionsdaten-Teilsätze sollen unter den vorstehend benutzten Begriff der CT-Projektionsdatensätze fallen), welche in Scanrichtung bzw. Zeilenrichtung (auch z-Richtung genannt) benachbart angeordnet sind, zusätzliche Projektionsdaten(-Teil)sätze auf Basis einer Interpolation eingefügt werden. An dieser Stelle ist anzumerken, dass ein Röntgendetektor zweidimensional ausgebildet ist und sowohl einander benachbarte Detektorzeilen in Scanrichtung aufweist als auch quer dazu Kanalreihen in Kanalrichtung aufweist. Ein Projektionsdaten(-Teil)satz umfasst nun vorzugsweise CT-Projektionsdaten, die einer solchen Kanalreihe oder Detektorzeile zugeordnet sind.

Eine Erhöhung der Auflösung von CT-Projektionsdaten kann nun nicht nur durch das Einfügen eines ganzen CT-Projektionsdatensatzes, welcher einem neuen Winkel zugeordnet ist, in die CT-Projektionsdaten erfolgen, sondern die Auflösung innerhalb der CT-Projektionsdatensätze kann ebenfalls erhöht werden, indem ebenfalls durch Interpolation anschaulich gesprochen Projektionsdaten zwischen den Projektionsdaten benachbarter Detektorzeilen und/oder Kanalreihen erzeugt und eingefügt werden.

Mithin kann eine niedrigere Detektorauflösung durch eine entsprechende Augmentierung von Projektionsdaten(-Teil)sätzen, welche Detektorzeilen oder quer zu den Detektorzeilen verlaufenden benachbarten Kanalreihen, die in Fächerrichtung verlaufen, zugeordnet sind, erfolgen.

Vorteilhaft kann bei gleichbleibender Bildqualität die Anzahl der von einem Untersuchungsobjekt aufgenommenen Projektionen reduziert werden und damit die Strahlenbelastung des Patienten proportional zu der Reduktion der aufgenommenen Projektionen reduziert werden. Alternativ kann die Bildqualität bei gleichbleibender Anzahl der aufgenommenen Projektionen und damit bei gleichbleibender Strahlenbelastung verbessert werden. Ein weiterer Vorteil des erfindungsgemäßen Verfahrens besteht darin, dass die Vervielfachung bzw. Augmentierung bereits im Projektionsdatenraum als vorgelagerter Schritt vor der eigentlichen Bildrekonstruktion erfolgt, der Schritt der Bildrekonstruktion also nicht modifiziert werden muss. Wie bereits erwähnt, kann mit dem erfindungsgemäßen Verfahren auch die Auflösung innerhalb einzelner Projektionen erhöht werden.

Bei dem erfindungsgemäßen Verfahren zum Generieren von CT-Bilddaten werden augmentierte CT-Projektionsdaten auf Basis des erfindungsgemäßen Verfahrens zur Generierung von augmentierten Projektionsdaten von einem Untersuchungsobjekt gewonnen. Weiterhin werden CT-Bilddaten auf Basis der augmentierten CT-Projektionsdaten rekonstruiert und gewonnen. Das erfindungsgemäße Verfahren zum Generieren von CT-Projektionsdaten von einem Untersuchungsobjekt teilt die Vorteile des erfindungsgemäßen Verfahrens zur Generierung von augmentierten Projektionsdaten von einem Untersuchungsobjekt.

Die erfindungsgemäße Projektionsdaten-Augmentierungseinrichtung umfasst eine Eingangsschnittstelle, welche dazu eingerichtet ist, unterabgetastete CT-Projektionsdaten von einem Untersuchungsobjekt, welche benachbarte CT-Projektionsdatensätze umfassen, zu empfangen.

Die erfindungsgemäße Projektionsdaten-Augmentierungseinrichtung umfasst auch eine Interpolationseinheit, welche dazu eingerichtet ist, synthetische CT-Projektionsdaten durch Anwendung eines auf einer Transformer-Architektur basierenden KI-basierten Modells auf die unterabgetasteten CT-Projektionsdaten KI-basiert zu generieren. Dabei werden zeitlich zwischen den benachbarten CT-Projektionsdatensätzen der unterabgetasteten CT-Projektionsdaten liegende zusätzliche CT-Projektionsdatensätze durch Interpolation zwischen den benachbarten CT-Projektionsdatensätzen gewonnen.

Die erfindungsgemäße Projektionsdaten-Augmentierungseinrichtung weist auch eine Augmentierungseinheit zum verschachtelten Kombinieren der unterabgetasteten CT-Projektionsdaten und der synthetischen CT-Projektionsdaten auf, wobei augmentierte CT-Projektionsdaten gewonnen werden. Die erfindungsgemäße Projektionsdaten-Augmentierungseinrichtung teilt die Vorteile des erfindungsgemäßen Verfahrens zur Generierung von augmentierten Projektionsdaten von einem Untersuchungsobjekt.

Diese Offenbarung betrifft ferner ein Verfahren zum Trainieren eines auf einer Transformer-Architektur basierenden KI-basierten Modells zur Generierung von augmentierten CT-Projektionsdaten von einem Untersuchungsobjekt werden Trainingsdaten erzeugt, welche als Eingabedaten unterabgetastete CT-Projektionsdaten von einem Untersuchungsobjekt aufweisen und als Referenz-Ergebnisdaten CT-Projektionsdaten mit einer vorbestimmten Auflösung, vorzugsweise der zu generierenden augmentierten CT-Projektionsdaten aufweisen. Die vorbestimmte Auflösung ist höher als die Auflösung der unterabgetasteten Projektionsdaten und ist gleich der Auflösung der augmentierten CT-Projektionsdaten. Wie bereits erläutert, betrifft die Auflösung sowohl eine Auflösung in Projektionsrichtung (unterschiedliche Winkel der Projektion) als auch in Detektorrichtung (welche in der Regel zweidimensional eine Zeilenrichtung und eine Kanalrichtung umfasst).

Die unterabgetasteten Projektionsdaten werden vorzugsweise durch "Herausschneiden" bzw. Entnahme aus voll abgetasteten CT-Projektionsdaten bzw. Referenz-Ergebnisdaten gewonnen, welche anschließend weiter als Zieldatensatz bzw. Referenz-Ergebnisdaten fungieren. Alternativ muss man echte Trainingsdatenpaare durch Aufnahme mit einer Scaneinheit eines CT-Systems mit einer entsprechend reduzierten Anzahl von Projektionen oder einer entsprechend reduzierten Auflösung innerhalb der einzelnen CT-Projektionen bzw. CT-Projektionsdatensätze erzeugen.

Anschließend wird das auf einer Transformer-Architektur basierende KI-basierte Modell durch Anwenden des auf einer Transformer-Architektur basierenden KI-basierten Modells auf die Eingabedaten der Trainingsdaten und durch Verarbeiten der dabei erzeugten Ergebnisdaten gemeinsam mit den Referenz-Ergebnisdaten, beispielsweise durch Vergleichen der generierten augmentierten CT-Projektionsdaten mit den Referenz-Ergebnisdaten, trainiert.

Schließlich wird das trainierte, auf einer Transformer-Architektur basierende KI-basierte Modell zur Generierung von augmentierten CT-Projektionsdaten von einem Untersuchungsobjekt bereitgestellt. Als Trainingsdaten können CT-Projektionsdaten von beliebigen CT-Aufnahmen mit hoher Winkelauflösung und/oder hoher Detektorauflösung verwendet werden, wobei für die Eingabedaten ein Teil der Projektionsdaten weggelassen wird und der vollständige Projektionsdatensatz für die Referenz-Ergebnisdaten genutzt wird. Neben CT-Projektionsdaten, welche durch Messungen gewonnen wurden, können auch simulierte CT-Projektionsdaten mit hoher Auflösung (eine "hohe Auflösung" kann sowohl in Winkelrichtung als auch in Detektorrichtung, insbesondere auch in Zeilenrichtung und in Kanalrichtung erzielt werden), welche zum Beispiel basierend auf mathematischen Phantomen gewonnen wurden, als Trainingsdaten eingesetzt werden. Dies hat den Vorteil, dass im Allgemeinen eine unbegrenzte Anzahl von Trainingsdaten zur Verfügung steht. Die Erzeugung von neuen Trainingsdaten kann auch während eines Trainings eines KI-basierten Modells erfolgen. Dabei können nach jeder Epoche des Trainings neue Trainingsdaten erzeugt werden, um die Qualität des Trainings zu verbessern.

Das erfindungsgemäße Computertomographiesystem weist eine Scaneinheit zum Erfassen von CT-Projektionsdaten von einem Untersuchungsobjekt und eine Steuereinrichtung zum Ansteuern der Scaneinheit und zum Generieren von Bilddaten auf Basis der erfassten CT-Projektionsdaten auf. Teil des erfindungsgemäßen Computertomographiesystems ist auch eine erfindungsgemäße Projektionsdaten-Augmentierungseinrichtung, welche dazu eingerichtet ist, auf Basis der erfassten CT-Projektionsdaten augmentierte CT-Projektionsdaten zu generieren. Das erfindungsgemäße Computertomographiesystem teil die Vorteile des erfindungsgemäßen Verfahrens zur Generierung von augmentierten CT-Projektionsdaten von einem Untersuchungsobjekt.

Als Eingabedaten werden die hochaufgelösten CT-Projektionsdaten bevorzugt einfach künstlich unterabgetastet, indem jede zweite bzw. n-te Zeile bzw. jeder zweiten bzw. n-te Projektionsdatensatz oder Projektions(-Teil)datensatz entnommen wird und den Eingabedaten zugeordnet wird. Als Referenz-Ergebnisdaten können dann entweder die Gesamtdaten oder die nicht den Eingabedaten zugeordneten CT-Projektionsdaten verwendet werden, je nachdem, ob die Referenz-Ergebnisdaten mit den synthetischen CT-Projektionsdaten oder den augmentierten CT-Projektionsdaten verglichen werden sollen. Im Fall der Nutzung eines neuronalen Netzwerks umfasst ein "Vergleich" eine Anwendung einer Fehlerfunktion auf die Ist-Ausgabe, also die ermittelten Ergebnisdaten und auf die Soll-Ausgabe, also die Referenz-Ergebnisdaten. Auf Basis des durch die Fehlerfunktion ermittelten Fehlers erfolgt dann eine Fehlerrückführung (im Englischen auch als Backpropagation bezeichnet) der Gewichte des neuronalen Netzwerks. Der Trainingsprozess, auch als "Lernen" bezeichnet, umfasst schließlich eine Minimierung der Fehlerfunktion, indem die Gewichte des neuronalen Netzwerks angepasst werden. Mithin kann auch ein zur Anwendung der Superresolution konzipiertes KI-basiertes Modell trainiert werden. Dabei wird zur Erzeugung der Trainingsdaten vorzugsweise das ursprünglich hochaufgelöste Sinogramm als Referenz-Projektionsdatensatz bzw. Referenz-Ergebnisdaten genutzt und der Eingabedatensatz wird vorzugsweise entlang der Detektor-Raumrichtung bzw. Detektorrichtung entsprechend verkleinert. Als Eingabedaten können auch in Detektorrichtung bzw. Detektor-Raumrichtung (also in Zeilenrichtung und / oder in Kanalrichtung) unterabgetastete Projektionsdaten verwendet werden, wobei als Referenz-Ergebnisdaten Projektionsdaten mit erhöhter Auflösung in Detektorrichtung verwendet werden können. Das Konzept der Superresolution ist in Zhang et al. "Deep Learning for Single Image Super-Resolution: A Brief Review", arXiv:1808.03344v3 [cs.CV] 12 Jul 2019 beschrieben. Hiermit wird der Inhalt dieser Schrift in die vorliegende Patentanmeldung durch Bezug darauf einbezogen.

Das erfindungsgemäße Computerprogrammprodukt weist Programmcodeabschnitte auf, mit denen alle Schritte des erfindungsgemäßen Verfahrens zur Generierung von augmentierten CT-Projektionsdaten von einem Untersuchungsobjekt oder des erfindungsgemäßen Verfahrens zum Generieren von CT-Bilddaten ausgeführt werden, wenn das Programm in einer Steuereinrichtung eines Computertomographiesystems ausgeführt wird.

Eine weitgehend softwaremäßige Realisierung hat den Vorteil, dass bereits Computertomographiesysteme oder deren Steuereinrichtungen auf einfache Weise durch ein Software-Update nachgerüstet werden können, um auf die erfindungsgemäße Weise zu arbeiten.

Ein Großteil der zuvor genannten Komponenten der erfindungsgemäßen Projektionsdaten-Augmentierungseinrichtung kann ganz oder teilweise in Form von Softwaremodulen in einem Prozessor eines entsprechenden Rechensystems realisiert werden, **z. B.** von einer Steuereinrichtung eines Computertomographiesystems oder einem Computer, der zur Steuerung eines solchen Systems genutzt wird. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um die Schritte des erfindungsgemäßen Verfahrens zur Generierung von augmentierten CT-Projektionsdaten von einem Untersuchungsobjekt oder die Schritte des erfindungsgemäßen Verfahrens zum Generieren von CT-Bilddaten auszuführen, wenn das Programm in dem Rechensystem ausgeführt wird. Ein solches Computerprogrammprodukt kann neben dem Computerprogramm gegebenenfalls zusätzliche Bestandteile wie **z. B.** eine Dokumentation und/oder zusätzliche Komponenten, auch Hardware-Komponenten, wie **z.B.** Hardware-Schlüssel (Dongles etc.), zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, **z. B.** ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Das Rechensystem kann **z. B.** hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

Wie bereits vorstehend kurz erläutert, kann ein CT-Projektionsdatensatz grundsätzlich einen der folgenden Datensatztypen umfassen:

- Projektionsdaten, welche Messdaten einer Detektorzeile eines Röntgendetektors eines CT-Systems, mit dem die Projektionsdaten erfasst wurden, umfassen,
- Projektionsdaten, welche Messdaten einer Detektorkanalreihe umfassen, wobei die Detektorkanalreihe quer zu den Detektorzeilen des Röntgendetektors verlaufen,
- Projektionsdaten, welche eine Linie eines Sinogramms, der ein Projektionswinkel zugeordnet ist, umfassen.

Ein solcher CT-Projektionsdatensatz kann also sowohl mit der zweidimensionalen Auflösung des Röntgendetektors korrespondieren als auch mit der Anzahl der Linien des Sinogramms der CT-Projektionsdaten. Alle drei Richtungen (Winkelrichtung, Zeilenrichtung, Kanalrichtung) beeinflussen die räumliche Auflösung der auf Basis der CT-Projektionsdaten rekonstruierten CT-Bilddaten. Es soll an dieser Stelle erwähnt werden, dass das Konzept des auf einer Transformer-Architektur basierten Modells sowohl für die Augmentierung der CT-Projektionsdaten in Winkelrichtung als auch für die Umsetzung der Superresolution, also das Erhöhen der (vorzugsweise zweidimensionalen) Auflösung einzelner Projektionen anwendbar ist.

Erfindungsgemäß umfasst ein CT-Projektionsdatensatz Projektionsdaten, welche einer Linie in einem Sinogramm, der ein Projektionswinkel zugeordnet ist, zugeordnet sind. Weiterhin werden bei dem Schritt des verschachtelten Kombinierens der unterabgetasteten CT-Projektionsdaten und der synthetischen CT-Projektionsdaten Zwischenwinkeln zwischen zwei benachbarten Winkeln der unterabgetasteten CT-Projektionsdaten zugeordnete zusätzliche CT-Projektionsdatensätze der synthetischen CT-Projektionsdaten zwischen den beiden benachbarten Winkeln zugeordneten CT-Projektionsdatensätzen der unterabgetasteten CT-Projektionsdaten eingeordnet, so dass eine Augmentierung in Winkelrichtung erfolgt. Anschaulich gesprochen, werden Lücken der unterabgetasteten Projektionsdaten in Winkelrichtung gefüllt, um die Auflösung der auf den CT-Projektionsdaten resultierenden CT-Bilddaten zu erhöhen und Artefakte der auf Basis der Projektionsdaten später zu rekonstruierenden CT-Bilddaten zu reduzieren und damit die Bildqualität zu verbessern.

In einer Ausgestaltung des erfindungsgemäßen Verfahrens zur Generierung von augmentierten CT-Projektionsdaten von einem Untersuchungsobjekt werden die Schritte ii) und iii), **d.h.** die Schritte zum KI-basierten Generieren von synthetischen CT-Projektionsdaten und zum verschachtelten Kombinieren der unterabgetasteten CT-Projektionsdaten und der synthetischen CT-Projektionsdaten mehrfach wiederholt, wobei bei der Wiederholung bei dem Schritt ii) anstatt der unterabgetasteten CT-Projektionsdaten die zuletzt gewonnenen augmentierten CT-Projektionsdaten für die Interpolation verwendet werden und bei dem Schritt iii) anstatt der unterabgetasteten CT-Projektionsdaten ebenfalls die zuletzt gewonnenen augmentierten CT-Projektionsdaten mit den synthetischen CT-Projektionsdaten verschachtelt kombiniert werden. Bei diesem sequenziellen Hintereinanderausführen des erfindungsgemäßen Verfahrens wird die Auflösung bei jedem Schleifendurchgang verdoppelt. Vorteilhaft wird der beschriebene vorteilhafte Effekt des erfindungsgemäßen Verfahrens zur Generierung von augmentierten Projektionsdaten von einem Untersuchungsobjekt besonders stark verstärkt.

In einer besonders bevorzugten Variante des erfindungsgemäßen Verfahrens zur Generierung von augmentierten Projektionsdaten von einem Untersuchungsobjekt werden die Schritte ii) und iii) zweimal wiederholt. Bei dieser Variante erfolgt eine Erhöhung der Auflösung M in Detektorrichtung um das Vierfache.

In einer alternativen Variante des erfindungsgemäßen Verfahrens wird der Schritt ii) parallel auf Basis der unterabgetasteten CT-Projektionsdaten durchgeführt und werden bei dem Schritt iii) die parallel gewonnenen synthetischen CT-Projektionsdaten mit den unterabgetasteten CT-Projektionsdaten verschachtelt kombiniert. Bei dieser Variante werden auf Basis der unterabgetasteten Projektionsdaten zwei oder mehr unterschiedliche synthetische CT-Projektionsdatensätze je benachbarten Winkelpaars gewonnen. Gemeinsam mit einem der unterabgetasteten CT-Projektionsdatensätze benachbarter Winkel werden die so gewonnenen synthetischen CT-Projektionsdatensätze zu einem augmentierten CT-Projektionsdatensatz verschachtelt kombiniert, so dass augmentierte CT-Projektionsdaten erzeugt werden, deren Auflösung von der Anzahl der parallel gewonnenen synthetischen CT-Projektionsdatensätze abhängt.

Besonders bevorzugt wird bei der vorstehend beschriebenen alternativen Variante der Schritt ii) zweifach parallel durchgeführt. D.h., es werden zwei synthetische CT-Projektionsdatensätze, denen unterschiedliche Projektionswinkel zugeordnet sind, erzeugt.

Alternativ kann der Schritt ii) auch mehr als zweifach parallel durchgeführt werden. Vorteilhaft wird die Datengrundlage für eine spätere Rekonstruktion verbreitert, so dass die Bildqualität der später rekonstruierten Bilddaten weiter erhöht wird.

Besonders bevorzugt wird die Augmentierung in Winkelrichtung mit einer Augmentierung in Detektorrichtung kombiniert, wobei die Augmentierung in Detektorrichtung durch Anwendung eines auf einem Superresolution-Ansatz KI-basierten Modells erfolgt. Vorteilhaft kann die Auflösung der Bilddaten in Detektorrichtung weiter verbessert werden. Diese Vorgehensweise ist insbesondere dann sehr effektiv, wenn die Datenübertragung der Projektionsdaten vom Röntgendetektor zur Steuereinrichtung stark eingeschränkt ist, weil dann die reduzierte Datenrate durch eine nachträgliche Augmentierung der Projektionsdaten auf Seiten der stationären Steuereinrichtung kompensiert werden kann.

Bevorzugt erfolgt die Anwendung eines auf einem Superresolution-Ansatz basierenden KI-basierten Modells auf die unterabgetasteten CT-Projektionsdaten vor dem Schritt ii), wobei höher aufgelöste unterabgetastete CT-Projektionsdaten gewonnen werden. Bei den Schritten ii) und iii) werden dann anstatt der weniger hoch aufgelösten unterabgetasteten CT-Projektionsdaten die höher aufgelösten unterabgetasteten CT-Projektionsdaten verwendet.

Alternativ erfolgt zunächst in Detektorrichtung eine Unterabtastung und es werden die Schritte ii) und iii) auf Basis der sowohl in Winkelrichtung (auch als Projektionsrichtung bezeichnet) als auch in Detektorrichtung unterabgetasteten CT-Projektionsdaten durchgeführt. Mithin werden die CT-Projektionsdaten anders als bei der vorstehend beschriebenen Variante zunächst in Winkelrichtung augmentiert. Anschließend wird die Unterabtastung in Detektorrichtung durch Anwendung des auf einem Superresolution-Ansatz KI-basierten Modells kompensiert. Es soll an dieser Stelle erwähnt werden, dass auch das Konzept der Superresolution durch ein auf einem Transformer-Netzwerk basierendes Modell implementiert werden kann und vorzugsweise implementiert wird.

Die beschriebene Kombination aus einer auf einem Transformeransatz basierenden Augmentierung von CT-Projektionsdaten in Winkelrichtung und einer auf Superresolution basierenden Augmentierung in Detektorraumrichtung kann bei Systemen mit reduzierter Anzahl an Detektorelementen sinnvoll sein oder bei Scan-Modi, bei denen ein Unterabtasten der gemessenen Daten bereits auf Detektorebene erfolgt und nur eine reduzierte Anzahl an Datensamples pro Projektion übermittelt wird.

Ein Grund für ein solches Unterabtasten kann zum Beispiel eine Bandbreitenlimitierung der Datenübertragungsstrecke zwischen dem Röntgendetektor und der Steuereinrichtung eines CT-Systems sein. Gerade im Bereich von neuartigen photonenzählenden Röntgendetektoren ist die benötigte Bandbreite zwischen dem Röntgendetektor und dem Bildrekonstruktionsrechner (welcher Teil der Steuereinrichtung ist) extrem hoch. Vor allem bei einer Anwendung der Scanmodi mit der höchsten räumlichen Auflösung (kleinste Detektorpixel) ist es bisher auf Grund der Bandbreitenlimitierung der Datenübertragungsstrecke nicht möglich, alle gemessenen Daten in voller Auflösung an den Bildrekonstruktionsrechner zu übertragen.

Um diese Limitation zu umgehen, werden bisher nur die Messdaten bzw. Projektionsdaten von einer der zwei Energieschwellen des Röntgendetektors in voller Auflösung übertragen. Die zweite Schwelle (oder ggf. weitere spektrale Schwellen, je nach technischer Ausprägung des Röntgendetektors) wird unterabgetastet und dann übertragen. D.h. es liegt nicht die gesamte spektrale Information am Bildrekonstruktionsrechner in der vollen Auflösung vor und spektrale Bilder können teilweise nur in geringerer Auflösung rekonstruiert werden. Hier ergibt sich eine weitere Anwendungsvariante der beschriebenen Anwendung eines Transformer-basierten und KI-basierten Modells bzw. eines auf dem Prinzip der Superresolution basierenden und auf KI basierten Modells. Neben den unterabgetasteten Daten der Schwelle 2 können auch die hochaufgelösten Daten der Schwelle 1 als Eingabedaten für das jeweilige Modell dienen. Aufgabe des Transformer-basierten Modells und/oder des auf der Superresolution basierenden Modells ist es dann, aus den beiden Eingabedatensätzen die Daten der Schwelle 2 in voller Auflösung zu erzeugen. Alternativ zum bisher verwendeten Unterabtasten der Detektorbilder wäre auch ein Unterabtasten in Projektionsrichtung bzw. Winkelrichtung vorstellbar, um die Datenrate zu reduzieren. Die oben beschriebenen Transformer-basierten Modelle und die auf dem Prinzip der Superresolution basierenden Modelle können auch in diesem Fall dazu verwendet werden, um die fehlenden Projektionen zu berechnen und dadurch die in den rekonstruierten Bildern erzielbare Auflösung zu erhöhen bzw. Artefakte zu reduzieren.

Mit der Projektionsrichtung ist die Winkelrichtung bzw. die Richtung der Gantryrotation gemeint. Typischerweise werden währen des Scans eine feste Anzahl von Projektionen pro 360° aufgenommen, die dann vom Röntgendetektor in den Rekonstruktionsrechner übertragen werden müssen. Verringert man die Anzahl der zu übertragenden Projektionen pro 360° verringert sich entsprechend auch die benötigte Bandbreite. Typischerweise hat man in der CT einen 2-dimensionalen Röntgendetektor mit einer bestimmten Anzahl von Zeilen in z-Richtung bzw. Zeilenrichtung und einer bestimmten Anzahl von Kanälen in der orthogonalen Richtung. Oft spricht man bei dieser Richtung auch von der Fächerrichtung.

Die Erfindung wird im Folgenden unter Hinweis auf die beigefügten Figuren anhand von Ausführungsbeispielen noch einmal näher erläutert. Es zeigen:

FIG 1 eine schematische Darstellung von Sinogrammen mit unterschiedlicher Abtastungsfrequenz und zugeordneten rekonstruierten Bilddaten,

FIG 2 eine schematische Darstellung der Grundstruktur einer Transformer-Architektur,

FIG 3 ein Schaubild, welches ein Verfahren zur Generierung von augmentierten CT-Projektionsdaten von einem Untersuchungsobjekt gemäß einem ersten Ausführungsbeispiel der Erfindung verdeutlicht,

FIG 4 ein Schaubild, welches ein Verfahren zur Generierung von augmentierten CT-Projektionsdaten von einem Untersuchungsobjekt mit sequenzieller Anwendung eines Transformer-Netzwerks gemäß einem zweiten Ausführungsbeispiel der Erfindung verdeutlicht,

FIG 5 ein Schaubild, welches ein Verfahren zur Generierung von augmentierten CT-Projektionsdaten von einem Untersuchungsobjekt mit paralleler Anwendung eines Transformer-Netzwerks gemäß einem dritten Ausführungsbeispiel der Erfindung verdeutlicht,

FIG 6 ein Schaubild, welches ein Verfahren zur Generierung von augmentierten CT-Projektionsdaten von einem Untersuchungsobjekt mit sequenzieller Anwendung eines Transformer-Netzwerks und eines Superresolution-Netzwerks (welches möglicherweise ebenfalls auf einer Transformer-Architektur **ba**siert) veranschaulicht,

FIG 7 ein Flussdiagramm, welches ein Verfahren zum Generieren von CT-Bilddaten gemäß einem Ausführungsbeispiel der Erfindung veranschaulicht,

FIG 8 ein Blockdiagramm, welches eine Projektionsdaten-Augmentierungseinrichtung gemäß einem Ausführungsbeispiel der Erfindung veranschaulicht,

FIG 9 eine schematische Darstellung eines Computertomographiesystems gemäß einem Ausführungbeispiel der Erfindung.

In FIG 1 ist eine schematische Darstellung 10 von vier Sinogrammen mit unterschiedlicher Abtastungsfrequenz und zugeordneten rekonstruierten Bilddaten gezeigt.

Ein erstes links in der Darstellung 10 gezeigtes Original-Sinogramm OS weist 360 Projektionen P auf. Darunter dargestellte rekonstruierte Bilddaten BD sollen als Referenzbild für die übrigen Bilddarstellungen dienen.

Ein zweites stark unterabgetastetes Sinogramm SS ist an zweiter Position von links in FIG 1 dargestellt und weist 90 Projektionen P auf. In den darunter angeordneten zugeordneten rekonstruierten Bilddaten sind sogenannte Streaking-Artefakte zu erkennen, die auf die stark ausgeprägte Unterabtastung zurückzuführen sind.

Ein drittes interpoliertes Sinogramm IS1 mit 180 Projektionen P, welches auf den 90 Projektionen P des zweiten stark unterabgetasteten Sinogramms SS basiert und durch eine Interpolation der 90 Projektionen P gewonnen wurde, ist an dritter Stelle von links in FIG 1 dargestellt. In den zugehörigen Bilddaten BD, welche unter dem Sinogramm IS1 gezeigt sind, sind im Vergleich zu dem an zweiter Stelle von links dargestellten Bild die Streaking-Artefakte verschwunden.

Ein viertes interpoliertes Sinogramm IS2 mit 360 Projektionen P, welches ebenfalls auf den 90 Projektionen P des zweiten stark unterabgetasteten Sinogramms SS basiert und durch eine zweifach ausgeführte sequenzielle Interpolation der 90 Projektionen P des zweiten stark unterabgetasteten Sinogramms SS gewonnen wurde, ist ganz rechts in FIG 1 gezeigt. Auch hier sind in den zugehörigen Bilddaten BD, welche unter dem Sinogramm IS2 gezeigt sind, die Streaking-Artefakte verschwunden.

In FIG 2 ist eine schematische Darstellung der Grundstruktur einer Transformer-Architektur 20 veranschaulicht. Die Transformer-Architektur 20 umfasst einen sogenannten Encoder EC, welcher in FIG 2 auf der linken Seite gezeigt ist, und einen Decoder DC, welcher in FIG 2 auf der rechten Seite gezeigt ist. Der Encoder EC umfasst K Schichten, welche jeweils zwei Unterschichten umfassen. Eine erste Unterschicht umfasst einen Multi-Kopf-Selbstaufmerksamkeitsmechanismus MHA, eine residuale Verbindung und eine Schichtnormalisierung RCN. Eine Aufmerksamkeitsfunktion kann als Zuordnung einer Abfrage und einer Reihe von Schlüssel-Wert-Paaren zu einer Ausgabe beschrieben werden, wobei die Abfrage, die Schlüssel, die Werte und die Ausgabe allesamt Vektoren sind. Die Ausgabe wird als gewichtete Summe der Werte berechnet, wobei die jedem Wert zugewiesene Gewichtung durch eine Kompatibilitätsfunktion der Abfrage mit dem zugeordneten Schlüssel berechnet wird.

Eine "Abfrage" ist eine Darstellung des aktuellen Eingabevektors, auf den sich das Modell konzentriert, und wird verwendet, um Aufmerksamkeitswerte zu berechnen. Der "Schlüssel" entspricht allen Eingabevektoren in der Eingabesequenz, auf die der aktuelle Eingabevektor achten könnte, und diese werden verwendet, um Kompatibilitätswerte mit der Abfrage zu berechnen. Der "Wert" stellt den eigentlichen Inhalt der Eingabevektoren in der Eingabesequenz dar, die gemäß den Aufmerksamkeitswerten gewichtet werden, um die endgültige Ausgabe zu erzeugen. Im Wesentlichen verwendet das Modell Abfragen und Schlüssel, um zu bestimmen, auf welche Werte (Eingabevektoren) in einem gegebenen Kontext geachtet werden sollte.

Die Multi-Head-Aufmerksamkeit ermöglicht es dem Modell, gemeinsam auf Informationen aus verschiedenen Darstellungs-Unterräumen an verschiedenen Positionen zuzugreifen. Bei einem einzigen Aufmerksamkeitskopf wird dies durch die Mittelwertbildung verhindert.

Mathematisch formuliert ergibt sich:

$$\text{MultiHead}(Q, K, V) = \text{Concat}(\text{head}_1, \ldots, \text{head}_h)W^O,$$

,

wobei $\text{head}_i = \text{Attention}(QW_i^Q, KW_i^K, VW_i^V)$,

wobei die Projektionen Parametermatrizen $W_i^Q \in R^{d_{model} \times d_k}$ $W_i^K \in R^{d_{model} \times d_k}$, $W_i^V \in R^{d_{model} \times d_v}$ und $W^O \in R^{d_v \times d_{model}}$ mit den Dimensionen $d_{model}$ und $d_k$ sind und MultiHead für einen "Multi-Kopf" und "Attention" für eine Aufmerksamkeitsfunktion stehen.

Aufgrund der reduzierten Abmessungen jedes Kopfes verringert sich der Gesamtrechenaufwand ähnlich einer Ein-Kopf-Aufmerksamkeit mit voller Dimensionalität.

Die zweite Unterschicht umfasst einen sogenannten Feed-Forward-Algorithmus FFN und eine residuale Verbindung und eine Schichtnormalisierung RCN.

Der Decoder DC (rechts im Bild) weist neben den bereits genannten Subschichten, welche bei dem Encoder EC zu finden sind, noch zusätzlich eine Subschicht mit einem maskierten Multi-Kopf-Selbstaufmerksamkeitsmechanismus MMHA und einer residualen Verbindung und einer Schichtnormalisierung RCN auf.

Am Eingang des Encoders EC und des Decoders DC wird jeweils ein sogenanntes Positionsenkodieren PE verwendet.

Da das Transformer-Modell keine Wiederholung und keine Faltung enthält, kann das Modell von der Positionskodierung Gebrauch machen. In der Reihenfolge der Sequenz müssen einige Informationen über die relative oder absolute Position in der Sequenz eingefügt werden.

Zu diesem Zweck werden "Positionskodierungen" PE zu den Eingabedaten an den Unterseiten der Encoder- und Decoderstapel hinzugefügt. Die Positionskodierungen haben die gleiche Dimension $d_{model}$ wie die Eingabedaten ED, so dass die beiden summiert werden können. Es gibt viele Möglichkeiten für Positionskodierungen, beispielsweise "gelernt" und "fixiert".

In den Encoder EC werden Eingangsdaten ED eingegeben und in den Decoder DC werden Ausgangsdaten AD eingegeben. Aus dem Decoder DC werden auch Ausgangsdaten AD ausgegeben.

Weitere Einzelheiten zur Transformer-Architektur sind in Vaswani A. et al., "Attention Is All You Need", 31st Conference on Neural Information Processing Systems (NIPS 2017), Long Beach, CA, USA beschrieben.

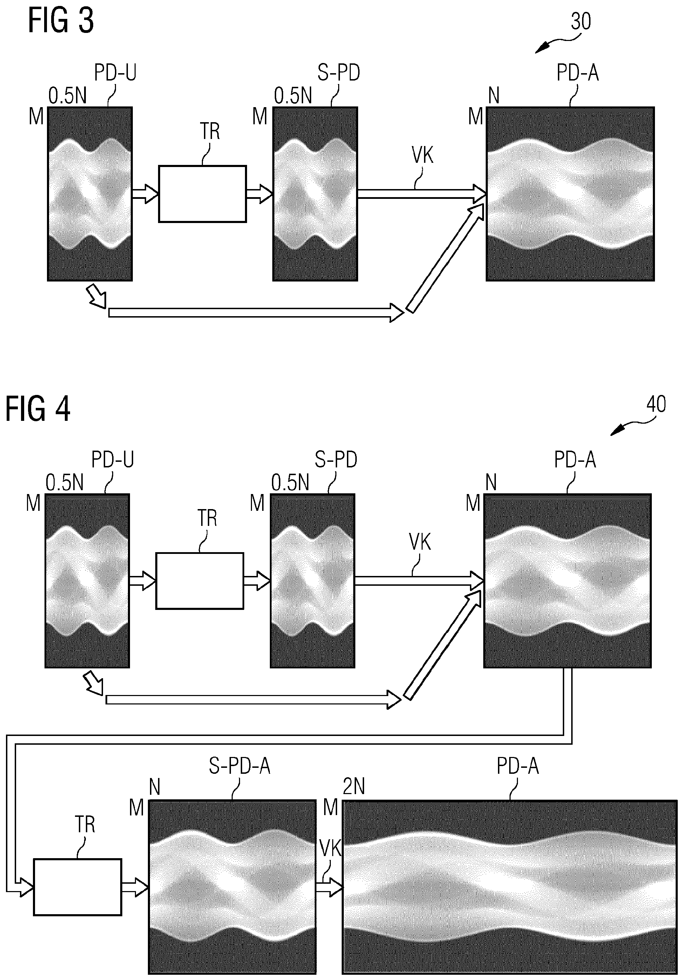

In FIG 3 ist ein Schaubild 30 gezeigt, welches ein Verfahren zur Generierung von augmentierten CT-Projektionsdaten von einem Untersuchungsobjekt gemäß einem ersten Ausführungsbeispiel der Erfindung verdeutlicht. Links in FIG 3 ist ein Sinogramm von unterabgetasteten Projektionsdaten PD-U gezeigt. Die Anzahl **M,** eine natürliche Zahl, gibt die Anzahl der in Detektorrichtung für die Generierung einer Projektion genutzten Einzelpixel des Detektors an. Die Anzahl 0.5 N gibt die Anzahl der Projektionen je 360°-Winkel an, wobei N und auch 0.5 N ebenfalls eine natürliche Zahl ist. In Projektionsrichtung bzw. Winkelrichtung erfolgt also in dem in FIG 3 dargestellten Beispiel eine 50-prozentige Unterabtastung. In FIG 3 ist außerdem ein Schritt TR symbolisiert, bei dem durch Anwendung eines auf einer Transformer-Architektur basierenden KI-basierten Modells auf die unterabgetasteten Projektionsdaten PD-U synthetische CT-Projektionsdaten S-PD ermittelt werden. Dabei werden zusätzliche CT-Projektionsdatensätze zwischen in Winkelrichtung aufeinanderfolgenden bzw. benachbarten CT-Projektionsdatensätzen der unterabgetasteten Projektionsdaten PD-U durch Interpolation zwischen den aufeinanderfolgenden bzw. einander benachbarten CT-Projektionsdatensätzen der unterabgetasteten Projektionsdaten PD-U ermittelt. Als "Projektionsdatensatz" soll hier eine Projektionszeile verstanden werden, die sich in FIG 3 in vertikaler Richtung erstreckt. In dem in FIG 3 gezeigten Ausführungsbeispiel umfassen die unterabgetasteten Projektionsdaten PD-U und die synthetischen CT-Projektionsdaten S-PD jeweils 0.5 N Zeilen und damit auch 0.5 N Projektionsdatensätze.

Weiterhin ist in FIG 3 auf der rechten Seite ein Schritt eines verschachtelten Kombinierens VK der unterabgetasteten CT-Projektionsdaten PD-U mit den synthetischen CT-Projektionsdaten S-PD gezeigt, wobei augmentierte CT-Projektionsdaten PD-A mit der doppelten Auflösung N der unterabgetasteten Projektionsdaten PD-U in Projektionsrichtung generiert werden.

In FIG 4 ist ein Schaubild 40 dargestellt, welches ein Verfahren zur Generierung von augmentierten CT-Projektionsdaten von einem Untersuchungsobjekt mit sequenzieller Anwendung eines Transformer-Netzwerks gemäß einem zweiten Ausführungsbeispiel der Erfindung verdeutlicht. Bei dem in FIG 4 dargestellten zweiten Ausführungsbeispiel wird das in FIG 3 bereits veranschaulichte Verfahren sequenziell zweimal hintereinander ausgeführt, wobei bei der zweiten Augmentierung synthetische augmentierte CT-Projektionsdaten S-PD-A mit im Vergleich zu den unterabgetasteten CT-Projektionsdaten PD-U doppelter Auflösung N in Projektionsrichtung bzw. Winkelrichtung erzeugt werden. Anschließend werden auf Basis der augmentierten CT-Projektionsdaten PD-A und der synthetischen augmentierten CT-Projektionsdaten S-PD-A durch eine verschachtelte Kombination VK wiederum augmentierte Projektionsdaten PD-A, aber nun mit erneut verdoppelter Auflösung, also einer Auflösung von 2N, in Winkelrichtung erzeugt.

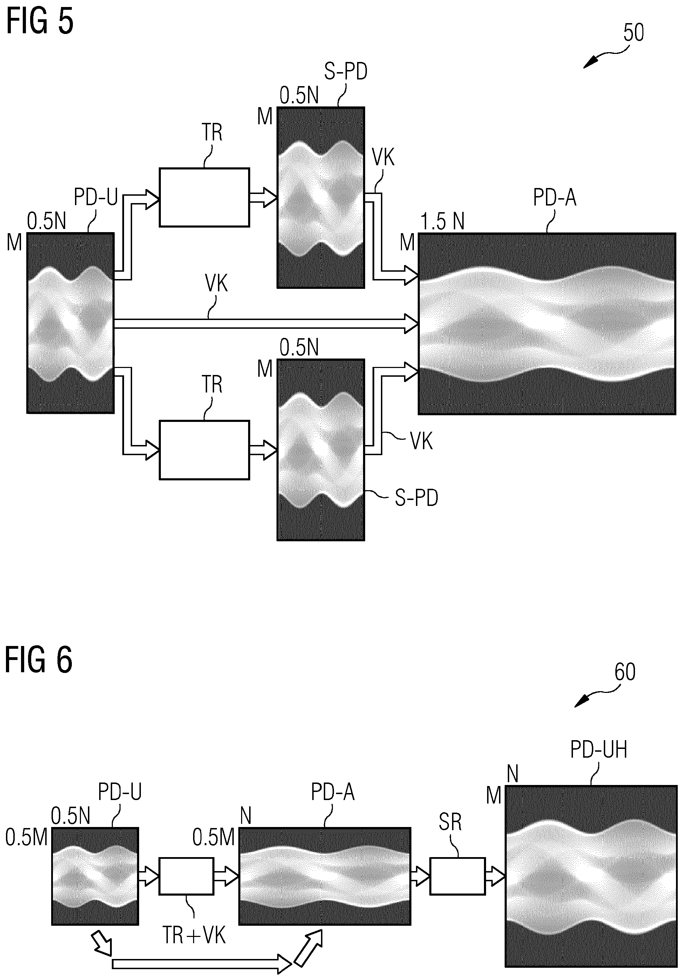

In FIG 5 ist ein Schaubild 50 gezeigt, welches ein Verfahren zur Generierung von augmentierten CT-Projektionsdaten von einem Untersuchungsobjekt mit paralleler Anwendung eines Transformer-Netzwerks gemäß einem dritten Ausführungsbeispiel der Erfindung verdeutlicht. Bei dem dritten Ausführungsbeispiel erfolgt anstatt einer sequenziellen doppelt ausgeführten Augmentierung eine parallel verdoppelt ausgeführte Augmentierung. Der Schritt des KI-basierten Generierens von synthetischen CT-Projektionsdaten S-PD wird also parallel ausgeführt bzw. auf ein und dieselben unterabgetasteten CT-Projektionsdaten PD-U parallel angewendet. Dabei werden die unterabgetasteten CT-Projektionsdaten PD-U jeweils unterschiedlich interpoliert, um zwei unterschiedliche Sätze von synthetischen CT-Projektionsdaten S-PD zu erhalten. Während zum Beispiel die ersten synthetischen CT-Projektionsdaten S-PD (oberer Zweig in FIG 5) jeweils einer ersten Zeile bzw. einem ersten Winkel zwischen zwei benachbarten Winkeln der unterabgetasteten Projektionsdaten PD-U entsprechen, entsprechen die zweiten synthetischen CT-Projektionsdaten S-PD (unterer Zweig in FIG 5) einem zweiten zu dem ersten Winkel versetzten Winkel zwischen den zwei benachbarten Winkeln der unterabgetasteten Projektionsdaten PD-U. Auf diese Weise können durch ein verschachteltes Kombinieren der unterabgetasteten CT-Projektionsdaten PD-U und der ersten und zweiten synthetischen CT-Projektionsdaten S-PD augmentierte CT-Projektionsdaten PD-A mit einer im Vergleich zu der Auflösung der unterabgetasteten CT-Projektionsdaten PD-U dreifachen Auflösung erzeugt werden. Die Auflösung in Projektionsrichtung (Winkelrichtung) entspricht also 1.5 N, wobei N gerade die durch die Scaneinheit ohne Interpolation erreichbare maximale Auflösung bzw. maximale Anzahl von Projektionen je 360°-Drehung des Röntgendetektors und der Röntgenquelle darstellen soll.

In FIG 6 ist ein Schaubild 60 gezeigt, welches ein Verfahren zur Generierung von augmentierten CT-Projektionsdaten von einem Untersuchungsobjekt mit sequenzieller Anwendung eines Transformer-Netzwerks und eines Superresolution-Netzwerks veranschaulicht. Anschaulich gesprochen, soll also bei dem in FIG 6 veranschaulichten Ausführungsbeispiel nicht nur die Auflösung in Projektionsrichtung erhöht werden, sondern auch in Detektorrichtung, wobei der Röntgendetektor der Einfachheit halber als eindimensional dargestellt ist. In der Realität ist die Sensorfläche eines Röntgendetektors zweidimensional. Der Wert M teilt sich dann auf in einen Wert M1 der Anzahl der Pixel in Zeilenrichtung und einen Wert M2 der Anzahl der Pixel in Kanalrichtung, wobei die Kanalrichtung quer zur Zeilenrichtung verläuft.

Die in FIG 6 veranschaulichte Variante kann sinnvoll sein, wenn die Menge der Datenübertragung der Projektionsdaten eingeschränkt ist. Beispielsweise wird dann, wie in FIG 6 gezeigt ist, nur jedes zweite Pixel der M Pixel in Detektorrichtung erfasst und dessen Signaldaten übertragen. Es findet quasi eine Unterabtastung sowohl in Detektorrichtung als auch in Projektionsrichtung statt, was in FIG 6 durch die Angaben "0.5 M" und "0.5 N" in vertikaler Richtung (Detektorrichtung) und horizontaler Richtung (Projektionsrichtung) des Sinogramms der unterabgetasteten CT-Projektionsdaten PD-U links im Bild veranschaulicht wird. Zunächst findet dann durch das bereits im Zusammenhang mit FIG 3 veranschaulichte Verfahren eine Erhöhung der Auflösung der unterabgetasteten Projektionsdaten PD-U in Projektionsrichtung statt, so dass das in FIG 6 in der Mitte gezeigte Sinogramm in Projektionsrichtung N Zeilen bzw. N Winkel umfasst. Anschließend erfolgt eine Anwendung eines KI-basierten Modells, welches auf einem für sogenannte Superresolution SR trainierten Netzwerk basiert. Auf diese Weise wird auch die Auflösung in Detektorrichtung von 0.5 M auf den Wert M erhöht, wobei ultrahochaufgelöste CT-Projektionsdaten PD-UH generiert werden.

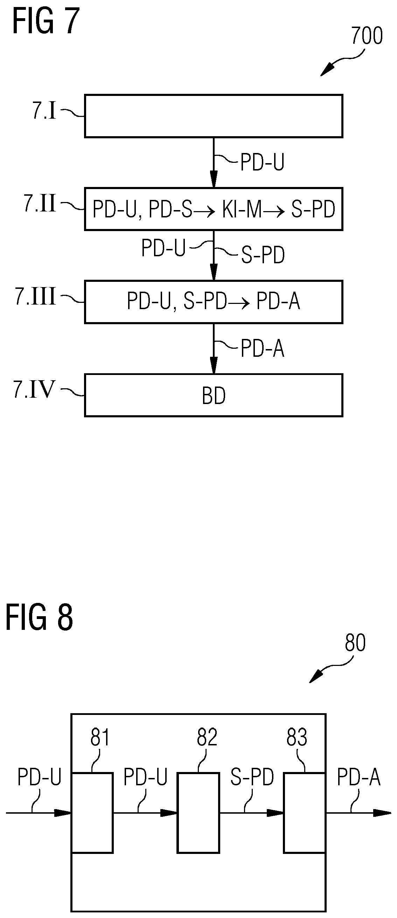

In FIG 7 ist ein Flussdiagramm gezeigt, welches ein Verfahren zum Generieren von CT-Bilddaten gemäß einem Ausführungsbeispiel der Erfindung veranschaulicht.

Bei den Schritten 7.I bis 7.III wird das im Zusammenhang mit FIG 3 bis FIG 6 ausführlich beschriebene Verfahren zur Generierung von augmentierten Projektionsdaten angewendet.

Im Detail wird bei dem Schritt 7.I eine Scaneinheit eines CT-Systems angesteuert und es werden unterabgetastete CT-Projektionsdaten PD-U von einem in der Scaneinheit befindlichen Untersuchungsobjekt O aufgenommen.

Bei dem Schritt 7.II wird ein auf einer Transformer-Architektur basierendes KI-basiertes Modell KI-M auf die unterabgetasteten CT-Projektionsdaten PD-U angewendet, wobei zwischen benachbarten CT-Projektionsdatensätzen PD-S der unterabgetasteten CT-Projektionsdaten PD-U liegende zusätzliche CT-Projektionsdatensätze durch Interpolation zwischen den benachbarten CT-Projektionsdatensätzen PD-S als synthetische CT-Projektionsdaten S-PD gewonnen werden.

Bei dem Schritt 7.III werden augmentierte CT-Projektionsdaten PD-A durch verschachteltes Kombinieren der bei dem Schritt 7.I gewonnenen unterabgetasteten CT-Projektionsdaten PD-U und der bei dem Schritt 7.II generierten synthetischen CT-Projektionsdaten S-PD erzeugt.

Schließlich werden bei dem Schritt 7.IV hochaufgelöste CT-Bilddaten BD auf Basis der augmentierten CT-Projektionsdaten PD-A rekonstruiert.

In FIG 8 ist ein Blockdiagramm gezeigt, welches eine Projektionsdaten-Augmentierungseinrichtung 80 gemäß einem Ausführungsbeispiel der Erfindung veranschaulicht. Die Projektionsdaten-Augmentierungseinrichtung 80 weist eine Eingangsschnittstelle 81 auf, welche dazu eingerichtet ist, unterabgetastete CT-Projektionsdaten PD-U von einem Untersuchungsobjekt O zu empfangen.

Teil der erfindungsgemäßen Projektionsdaten-Augmentierungseinrichtung 80 ist auch eine Interpolationseinheit 82. Die Interpolationseinheit 82 ist dazu eingerichtet, synthetische CT-Projektionsdaten S-PD durch Anwendung eines auf einer Transformer-Architektur basierenden KI-basierten Modells KI-M auf die unterabgetasteten CT-Projektionsdaten PD-U zu generieren. Dabei werden zwischen benachbarten CT-Projektionsdatensätzen PD-S der unterabgetasteten CT-Projektionsdaten PD-U liegende, zusätzliche CT-Projektionsdatensätze durch Interpolation zwischen den benachbarten CT-Projektionsdatensätzen PD-S gewonnen.

Die Projektionsdaten-Augmentierungseinrichtung 80 umfasst auch eine Augmentierungseinheit 83 zum verschachtelten Kombinieren der unterabgetasteten CT-Projektionsdaten PD-U und der synthetischen CT-Projektionsdaten S-PD, wobei augmentierte CT-Projektionsdaten PD-A gewonnen werden.

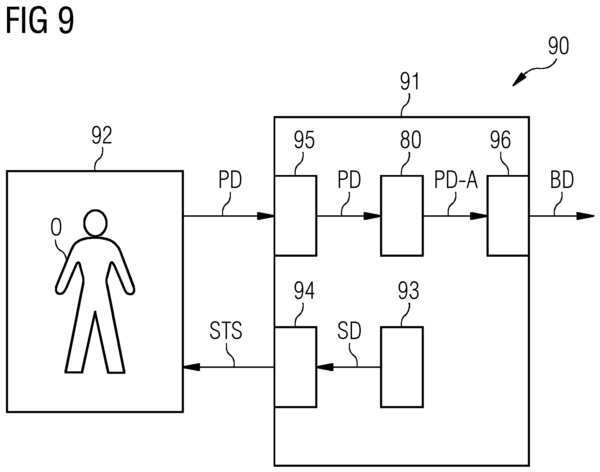

In FIG 9 ist eine schematische Darstellung eines Computertomographiesystems 90 gemäß einem Ausführungsbeispiel der Erfindung gezeigt.

Das Computertomographiesystem 90 weist eine Steuereinrichtung 91 und eine von der Steuereinrichtung 91 angesteuerte Scaneinheit 92 auf. Die Steuereinrichtung 91 weist eine Steuereinheit 93 zum Erzeugen von Steuerdaten SD und eine Steuerschnittstelle 94 zum Übermitteln von Steuersignalen STS, welche auf Basis der Steuerdaten SD erzeugt werden, an die Scaneinheit 92 auf. Teil der Steuereinrichtung 91 ist auch eine Eingangsschnittstelle 95, welche dazu eingerichtet ist, CT-Projektionsdaten PD von der Scaneinheit 92 zu empfangen. Die CT-Projektionsdaten PD werden an eine Projektionsdaten-Augmentierungseinrichtung 80 übermittelt, welche dazu eingerichtet ist, auf Basis der CT-Projektionsdaten PD augmentierte CT-Projektionsdaten PD-A mit erhöhter Auflösung zu erzeugen.

Die augmentierten Projektionsdaten PD-A werden an eine Rekonstruktionseinheit 96 übermittelt, welche ebenfalls Teil der Steuereinrichtung 91 ist und dazu eingerichtet ist, Bilddaten von einem in der Scaneinheit 92 liegenden Untersuchungsobjekt O zu rekonstruieren.

Dabei ist darauf hinzuweisen, dass die Merkmale sämtlicher Ausführungsbeispiele oder in Figuren offenbarter Weiterbildungen in beliebiger Kombination verwendet werden können.

Es wird abschließend noch einmal darauf hingewiesen, dass es sich bei den zuvor beschriebenen detaillierten Verfahren und Aufbauten um Ausführungsbeispiele handelt und dass das Grundprinzip auch in weiten Bereichen vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

**Patentansprüche**

1. Verfahren zur Generierung von augmentierten CT-Projektionsdaten (PD-A) von einem Untersuchungsobjekt (O), aufweisend die Schritte:

   i) Empfangen von unterabgetasteten CT-Projektionsdaten (PD-U), welche benachbarte CT-Projektionsdatensätze (PD-S) umfassen, von dem Untersuchungsobjekt (O), wobei ein CT-Projektionsdatensatz (PD-S) Projektionsdaten umfasst, welche einer Linie in einem Sinogramm, der ein Projektionswinkel zugeordnet ist, zugeordnet sind,

   ii) KI-basiertes Generieren von synthetischen CT-Projektionsdaten (S-PD) durch Anwendung eines auf einer Transformer-Architektur basierenden KI-basierten Modells (KI-M) auf die unterabgetasteten CT-Projektionsdaten (PD-U), wobei zwischen den benachbarten CT-Projektionsdatensätzen (PD-S) der unterabgetasteten CT-Projektionsdaten (PD-U) liegende zusätzliche CT-Projektionsdatensätze durch Interpolation zwischen den benachbarten CT-Projektionsdatensätzen (PD-S) gewonnen werden,

   iii) verschachteltes Kombinieren der unterabgetasteten CT-Projektionsdaten (PD-U) und der synthetischen CT-Projektionsdaten (S-PD), wobei augmentierte CT-Projektionsdaten (PD-A) gewonnen werden, wobei Zwischenwinkeln zwischen zwei benachbarten Winkeln der unterabgetasteten CT-Projektionsdaten (PD-U) zugeordnete zusätzliche CT-Projektionsdatensätze der synthetischen CT-Projektionsdaten (S-PD) zwischen den zwei benachbarten Winkeln zugeordneten CT-Projektionsdatensätzen (PD-S) der unterabgetasteten CT-Projektionsdaten (PD-U) eingeordnet werden, so dass eine Augmentierung in Winkelrichtung erfolgt.

2. Verfahren nach Anspruch **1,** wobei die Schritte ii) und iii) mehrfach wiederholt werden, wobei bei der Wiederholung bei dem Schritt ii) anstatt der unterabgetasteten CT-Projektionsdaten (PD-U) die zuletzt gewonnenen augmentierten CT-Projektionsdaten (PD-A) für die Interpolation verwendet werden und bei dem Schritt iii) anstatt der unterabgetasteten CT-Projektionsdaten (PD-U) ebenfalls die zuletzt gewonnenen augmentierten CT-Projektionsdaten (PD-A) mit den synthetischen CT-Projektionsdaten (S-PD) verschachtelt kombiniert werden.

3. Verfahren nach Anspruch **2,** wobei die Schritte ii) und iii) zweifach wiederholt werden.

4. Verfahren nach einem der vorstehenden Ansprüche, wobei der Schritt ii) parallel auf Basis der unterabgetasteten CT-Projektionsdaten (PD-U) durchgeführt wird und bei dem Schritt iii) die parallel gewonnenen synthetischen CT-Projektionsdaten (S-PD) mit den unterabgetasteten CT-Projektionsdaten (PD-U) verschachtelt kombiniert werden.

5. Verfahren nach Anspruch **4,** wobei der Schritt ii) zweifach parallel durchgeführt wird.

6. Verfahren nach Anspruch **4,** wobei der Schritt ii) mehr als zweifach parallel durchgeführt wird.

7. Verfahren nach einem der vorstehenden Ansprüche, wobei eine Augmentierung in Winkelrichtung mit einer Augmentierung in Detektorrichtung kombiniert wird, wobei die Augmentierung in Detektorrichtung durch Anwendung eines auf einem Superresolution-Ansatz basierenden KI-basierten Modells (KI-M) erfolgt.

8. Verfahren zum Generieren von CT-Bilddaten (BD), aufweisend die Schritte:

   - Gewinnen von augmentierten CT-Projektionsdaten (PD-A) auf Basis des Verfahrens nach einem der Ansprüche 1 bis **7,**
   - Rekonstruieren von CT-Bilddaten (BD) auf Basis der augmentierten CT-Projektionsdaten (PD-A).

9. Projektionsdaten-Augmentierungseinrichtung (80), aufweisend:

   - eine Eingangsschnittstelle (81) zum Empfangen von unterabgetasteten CT-Projektionsdaten (PD-U), welche benachbarte CT-Projektionsdatensätze (PD-S) umfassen, von einem Untersuchungsobjekt (O), wobei ein CT-Projektionsdatensatz (PD-S) Projektionsdaten umfasst, welche einer Linie in einem Sinogramm, der ein Projektionswinkel zugeordnet ist, zugeordnet sind,
   - eine Interpolationseinheit (82) zum KI-basierten Generieren von synthetischen CT-Projektionsdaten (S-PD) durch Anwendung eines auf einer Transformer-Architektur basierenden KI-basierten Modells (KI-M) auf die unterabgetasteten CT-Projektionsdaten (PD-U), wobei zwischen den benachbarten CT-Projektionsdatensätzen (PD-S) der unterabgetasteten CT-Projektionsdaten (PD-U) liegende zusätzliche CT-Projektionsdatensätze durch Interpolation zwischen den benachbarten CT-Projektionsdatensätzen (PD-S) gewonnen werden,
   - eine Augmentierungseinheit (83) zum verschachtelten Kombinieren der unterabgetasteten CT-Projektionsdaten (PD-U) und der synthetischen CT-Projektionsdaten (S-PD), wobei augmentierte CT-Projektionsdaten (PD-A) gewonnen werden, indem Zwischenwinkeln zwischen zwei benachbarten Winkeln der unterabgetasteten CT-Projektionsdaten (PD-U) zugeordnete zusätzliche CT-Projektionsdatensätze der synthetischen CT-Projektionsdaten (S-PD) zwischen den zwei benachbarten Winkeln zugeordneten CT-Projektionsdatensätzen (PD-S) der unterabgetasteten CT-Projektionsdaten (PD-U) eingeordnet werden, so dass eine Augmentierung in Winkelrichtung erfolgt.

10. Computertomographiesystem (90), aufweisend:

    - eine Scaneinheit (92) zum Erfassen von CT-Projektionsdaten (PD) von einem Untersuchungsobjekt (O),
    - eine Steuereinrichtung (91) zum Ansteuern der Scaneinheit (92) und zum Generieren von Bilddaten (BD) auf Basis der CT-Projektionsdaten (PD),
    - eine Projektionsdaten-Augmentierungseinrichtung (80) nach Anspruch 9.

11. Computerprogrammprodukt mit einem Computerprogramm, welches direkt in einen Speicher einer Steuereinrichtung (91) eines Computertomographiesystems (90) ladbar ist, mit Programmcodeabschnitten, um alle Schritte eines Verfahrens nach einem der Ansprüche 1 bis 8 auszuführen, wenn das Programm in der Steuereinrichtung (91) ausgeführt wird.

12. Computerlesbares Medium, auf welchem von einer Rechnereinheit ausführbare Programmabschnitte gespeichert sind, um die Schritte eines Verfahrens nach einem der Ansprüche 1 bis 8 auszuführen, wenn die Programmabschnitte von der Rechnereinheit ausgeführt werden.


**Claims**

1. Method for generating augmented CT projection data (PD-A) from an examination object (O), having the steps:

   i) receiving undersampled CT projection data (PD-U), which comprises adjacent CT projection data sets (PD-S), from the examination object (O), wherein a CT projection data set (PD-S) comprises projection data, which is assigned to a line in a sinogram assigned to a projection angle,
   ii) AI-based generation of synthetic CT projection data (S-PD) by way of application of an AI-based model (KI-M) based on a transformer architecture to the undersampled CT projection data (PD-U), wherein additional CT projection data sets located between the adjacent CT projection data sets (PD-S) of the undersampled CT projection data (PD-U) are obtained by way of interpolation between the adjacent CT projection data sets (PD-S),
   iii) interleaved combination of the undersampled CT projection data (PD-U) and the synthetic CT projection data (S-PD), wherein augmented CT projection data (PD-A) is obtained, wherein additional CT projection data sets of the synthetic CT projection data (S-PD) assigned to intermediate angles between two adjacent angles of the undersampled CT projection data (PD-U) are integrated between CT projection data sets (PD-S) of the undersampled CT projection data (PD-U) assigned to the two adjacent angles, enabling an augmentation in the angular direction to take place.

2. Method according to claim 1, wherein steps ii) and iii) are repeated multiple times, wherein, during the repetition, in step ii), the most recently obtained augmented CT projection data (PD-A) is used for the interpolation instead of the undersampled CT projection data (PD-U) and in step iii), instead of the undersampled CT projection data (PD-U), the most recently obtained augmented CT projection data (PD-A) is likewise combined in an interleaved manner with the synthetic CT projection data (S-PD).

3. Method according to claim 2, wherein steps ii) and iii) are repeated twice.

4. Method according to one of the preceding claims, wherein step ii) is performed in parallel on the basis of the undersampled CT projection data (PD-U) and in step iii) the synthetic CT projection data (S-PD) obtained in parallel is combined in an interleaved manner with the undersampled CT projection data (PD-U).

5. Method according to claim 4, wherein step ii) is performed twice in parallel.

6. Method according to claim 4, wherein step ii) is performed more than twice in parallel.

7. Method according to one of the preceding claims, wherein an augmentation in the angular direction is combined with an augmentation in the detector direction, wherein the augmentation in the detector direction takes place using an AI-based model (KI-M) based on a super resolution approach.

8. Method for generating CT image data (BD), having the steps:

   - obtaining augmented CT projection data (PD-A) based on the method according to one of claims 1 to 7,
   - reconstructing CT image data (BD) based on the augmented CT projection data (PD-A).

9. Projection data augmentation facility (80), having:

   - an input interface (81) for receiving undersampled CT projection data (PD-U) which comprises adjacent CT projection data sets (PD-S), from an examination object (O), wherein a CT projection data set (PD-S) comprises projection data, which is assigned to a line in a sinogram assigned to a projection angle,
   - an interpolation unit (82) for AI-based generation of synthetic CT projection data (S-PD) by way of application of an AI-based model (KI-M) based on a transformer architecture to the undersampled CT projection data (PD-U), wherein additional CT projection data sets located between the adjacent CT projection data sets (PD-S) of the undersampled CT projection data (PD-U) are obtained by way of interpolation between the adjacent CT projection data sets (PD-S),
   - an augmentation unit (83) for interleaved combination of the undersampled CT projection data (PD-U) and the synthetic CT projection data (S-PD), wherein augmented CT projection data (PD-A) is obtained, in that additional CT projection data sets of the synthetic CT projection data (S-PD) assigned to intermediate angles between two adjacent angles of the undersampled CT projection data (PD-U) are integrated between CT projection data sets (PD-S) of the undersampled CT projection data (PD-U) assigned to the two adjacent angles, enabling an augmentation in the angular direction to take place.

10. Computed tomography system (90), having:

    - a scanning unit (92) for acquiring CT projection data (PD) from an examination object (O),
    - a control facility (91) for controlling the scanning unit (92) and for generating image data (BD) based on the CT projection data (PD),
    - a projection data augmentation facility (80) according to claim 9.

11. Computer program product with a computer program, which can be loaded directly into a memory of a control facility (91) of a computed tomography system (90), with program code sections for carrying out all steps of a method according to one of claims 1 to 8 when the program is executed in the control facility (91).

12. Computer-readable medium, on which program sections which can be executed by a computer unit are stored for carrying out the steps of a method according to one of claims 1 to 8, when the program sections are executed by the computer unit.

**Revendications**

1. Procédé de création de données (PD-A) augmentées de projection CT d'un objet (O) à examiner, comportant les stades :

   i) réception de données (PD-U) sous-échantillonnées de projection CT, qui comprennent des ensembles (PD-S) voisins de données de projection CT de l'objet (O) à examiner, dans lequel un ensemble (PD-S) de données de projection CT comprend des données de projection, qui sont affectées à une ligne d'un sinogramme, qui est affecté à un angle de projection,

   ii) création reposant sur l'IA de données (S-PD) synthétiques de projection CT par application d'un modèle (KI-M) reposant sur l'IA sur la base d'une architecture de transformation aux données (PD-U) sous-échantillonnées de projection CT, dans lequel on obtient, par interpolation entre les ensembles (PD-S) voisins de données de projection CT, des ensembles supplémentaires de données de projection CT se trouvant entre les ensembles (PD-S) voisins de données de projection CT des données (PD-U) sous-échantillonnées de projection CT,

   iii) combinaison entrelacée des données (PD-U) sous-échantillonnées de projection CT et des données (S-PD) synthétiques de projection CT, dans lequel on obtient des données (PD-A) augmentées de projection CT, dans lequel on classe des ensembles supplémentaires de données de projection CT des données (S-PD) synthétiques de projection CT, affectées à des angles intermédiaires entre deux angles voisins des données (PD-U) de projection CT sous-échantillonnées, entre les ensembles (PD-S) de données de projection CT des données (PD-U) de projection CT sous-échantillonnées, affectées à deux angles voisins, de manière à obtenir une augmentation dans la direction angulaire.

2. Procédé suivant la revendication 1, dans lequel on répète plusieurs fois les stades ii) et iii), dans lequel, lors de la répétition dans le stade ii), on utilise, pour l'interpolation, au lieu des données (P-DU) de projection CT sous-échantillonnées les données (PD-A) augmentées obtenues en dernier de projection CT et dans le stade iii) on combine, de manière entrelacée, au lieu des données (PD-U) sous-échantillonnées de projection CT, également les données (PD-A) augmentées obtenues en dernier de projection CT aux données (S-PD) synthétiques de projection CT.

3. Procédé suivant la revendication 2, dans lequel on répète deux fois les stades ii) et iii).

4. Procédé suivant l'une des revendications précédentes, dans lequel on effectue les stades ii) parallèlement sur la base des données (PD-U) sous-échantillonnées de projection CT et dans le stade iii) on combine avec entrelacement les données (S-PD) synthétiques obtenues en parallèle de projection CT aux données (PD-U) sous-échantillonnées de projection CT.

5. Procédé suivant la revendication 4, dans lequel on effectue le stade ii) en parallèle deux fois.

6. Procédé suivant la revendication 4, dans lequel on effectue le stade ii) en parallèle plus de deux fois.

7. Procédé suivant l'une des revendications précédentes, dans lequel on combine une augmentation dans la direction angulaire à une augmentation dans une direction de détecteur, dans lequel l'augmentation dans la direction du détecteur s'effectue par application d'un modèle (KI-M) reposant sur l'IA sur la base d'une architecture de super résolution.

8. Procédé de création de données (BD) d'image CT, comprenant les stades :

   - obtention de données (PD-A) augmentées de projection CT sur la base du procédé suivant l'une des revendications 1 à 7,

   - reconstruction de données (BD) d'image CT sur la base des données (PD-A) augmentées de projection CT.

9. Dispositif (80) d'augmentation de données de projection, comportant :

   - une interface (81) d'entrée pour la réception de données (PD-U) sous-échantillonnées de projection CT, qui comprennent des ensembles (PD-S) voisins de données de projection CT de l'objet (O) à examiner, dans lequel un ensemble (PD-S) de données de projection CT comprend des données de projection, qui sont affectées à une ligne d'un sinogramme, qui est affecté à un angle de projection,

   - une unité (82) d'interpolation pour la création reposant sur l'IA de données (S-PD) synthétiques de projection CT par application d'un modèle (KI-M), reposant sur l'IA sur la base d'une architecture de transformation, aux données (PD-U) sous-échantillonnées de projection CT, dans lequel on obtient, par interpolation entre les ensembles (PD-S) voisins de données de projection CT, des ensembles supplémentaires de données de projection CT se trouvant entre les ensembles (PD-S) voisins de données de projection CT des données (PD-U) sous-échantillonnées de projection CT,

- une unité (83) d'augmentation pour la combinaison avec entrelacement des données (PD-U) sous-échantillonnées de projection CT et des données (S-PD) synthétiques de projection CT, dans lequel on obtient des données (PD-A) augmentées de projection CT, dans lequel on classe des ensembles supplémentaires de données de projection CT des données (S-PD) synthétiques de projection CT, affectées à des angles intermédiaires entre deux angles voisins des données (PD-U) de projection CT sous-échantillonnées, entre les ensembles (PD-S) de données de projection CT des données (PD-U) de projection CT sous-échantillonnées, affectées à deux angles voisins, de manière à obtenir une augmentation dans la direction angulaire.

10. Système (90) de tomodensitométrie assisté par ordinateur, comportant :

- une unité (92) de balayage pour la détection de données (PD) de projection CT d'un objet (O) à examiner,
- un dispositif (91) de commande pour la commande de l'unité (92) de balayage et pour la création de données (BD) d'image sur la base des données (PD) de projection CT,
- un dispositif (80) d'augmentation de données de projection suivant la revendication 9.

11. Produit de programme d'ordinateur comprenant un programme d'ordinateur, qui peut être chargé directement dans la mémoire d'un dispositif (91) de commande d'un système (90) de tomodensitométrie assisté par ordinateur, comprenant des parties de code de programme pour exécuter tous les stades du procédé suivant l'une des revendications 1 à 8, lorsque le programme est exécuté dans le dispositif (91) de commande.

12. Support déchiffrable par ordinateur, sur lequel sont mises en mémoire des parties de programme pouvant être exécutées par une unité informatique, afin d'exécuter les stades d'un procédé suivant l'une des revendications 1 à 8, lorsque les parties de programme sont exécutées par l'unité informatique.

## FIG 1

10

| OS 360 P | SS 90 P | IS1 180 P | IS2 360 P |
|----------|---------|-----------|-----------|

BD     BD     BD     BD

## FIG 2

20

DC

AD

RCN

FFN

RCN

MHA

RCN

MMHA

Kx

PE — AD — PE

EC

RCN

FFN

RCN

MHA

Kx

PE — ED

# FIG 3

30

PD-U

S-PD

PD-A

TR

VK

# FIG 4

40

PD-U

S-PD

PD-A

TR

VK

S-PD-A

PD-A

TR

VK

# FIG 5

50

# FIG 6

60

FIG 7

700

7.I

PD-U

7.II  PD-U, PD-S → KI-M → S-PD

PD-U  S-PD

7.III  PD-U, S-PD → PD-A

PD-A

7.IV  BD

FIG 8

80

81    82    83

PD-U  PD-U  S-PD  PD-A

FIG 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KIM et al.** Image enhancement for computed tomography using directional interpolation for sparsely-sampled sinogram. *Optik*, 2018, vol. 166 (2018), 227-235
- **LEE et al.** Deep-Neural-Network-Based Sinogram Synthesis for Sparse-View CT Image Reconstruction. *IEEE Transactions on Radiation and Plasma Medical Sciences*, March 2019, vol. 4 (2)
- **CHAO et al.** Sparse-View Cone Beam CT Reconstruction using dual CNNs in Projection Domain and Image Domain. *Neurocomputing*, 2022, vol. 493, 536-547

- **AMOGH SUBBAKRISHNA et al.** Sinogram Domain Angular Upsampling of Sparse-View Micro-CT with Dense Residual Hierarchical Transformer and Noise-Aware Loss. *bioRxiv*, 12 May 2023
- **CHRISTIANSEN C** ; **ZENG GL**. Sinogram Interpolation Inspired by Single-Image Super Resolution. *Journal of biotechnology and its applications 2023*, 15 June 2023, vol. 2 (1)
- **VASWANI A. et al.** Attention Is All You Need. *31st Conference on Neural Information Processing Systems (NIPS 2017), Long Beach, CA, USA*
- **ZHANG et al.** Deep Learning for Single Image Super-Resolution: A Brief Review. *arXiv:1808.03344v3 [cs.CV*, 12 July 2019